# FASCICULE DE BREVET EUROPEEN

(11) **EP 0 618 793 B1**
(45) Date de publication et mention de la délivrance du brevet: **02.05.1997**
(21) Numéro de dépôt: 93924650.0
(22) Date de dépôt: 28.10.1993
(51) Int. Cl.: A61K 7/06

(54) **COMPOSITION POUR LA FIXATION DE LA CHEVELURE**
HAARFESTIGUNGSMITTEL
HAIR-SETTING COMPOSITION

(30) Priorité: 28.10.1992 FR 9212872
(43) Date de publication de la demande: 12.10.1994
(73) Titulaire: L'OREAL, 75008 Paris (FR)
(72) Inventeur: MONDET, Jean, F-93700 Drancy (FR); STURLA, Jean-Michel, F-92150 Suresnes (FR); LION, Bertrand, F-93190 Livry-Gargan (FR); DUPUIS, Christine, F-75018 Paris (FR); CAZENEUVE, Colette, F-75015 Paris (FR)
(74) Mandataire: Stalla-Bourdillon, Bernard
(86) Numéro de dépôt international: FR9301063
(87) Numéro de publication internationale: WO9409749

(56) Documents cités:
- EP-A- 0 418 676
- FR-A- 2 439 798
- US-A- 4 960 814

## Description

La présente invention a pour objet une composition cosmétique aqueuse pour la fixation des cheveux contenant une dispersion aqueuse de particules d'un polymère filmogène et d'au moins un agent plastifiant.

Plus particulièrement, la présente invention a pour objet des compositions capillaires sous forme d'une laque aérosol, d'une lotion de fixation, ou de mise en plis ou encore d'une mousse de coiffage.

Depuis quelques années un intérêt tout particulier s'est manifesté pour la réalisation de compositions cosmétiques capillaires aqueuses. En effet, l'emploi d'alcool tel que l'éthanol ou l'isopropanol, seul ou en mélange avec une faible proportion d'eau, peut présenter certains inconvénients, notamment une augmentation de l'inflammabilité lorsque la composition est sous forme d'une laque aérosol.

Par ailleurs, pour des raisons écologiques en vue de préserver notamment la couche d'ozone la tendance actuelle est de remplacer dans les aérosols les hydrocarbures fluorochlorés de type "FREON" par des propulseurs moins toxiques tels que les hydrocarbures, l'air comprimé ou le diméthyléther.

Ces différentes exigences quant à la nature du véhicule et de l'agent propulseur sont extrêmement difficiles à concilier compte-tenu des nombreux facteurs entrant en jeu.

Ainsi, si la plupart des polymères filmogènes hydrosolubles peuvent en solution dans l'eau, éventuellement en présence d'un agent plastifiant, conduire à l'obtention d'une laque aérosol en présence d'un agent propulseur inerte tel que le diméthyléther, une telle laque souffre cependant de deux inconvénients majeurs.

Le premier est le faible pouvoir de fixation de la chevelure dans la mesure où l'on ne peut employer qu'une faible concentration en résine filmogène (<10%).

En effet une augmentation de la concentration a pour conséquence un accroissement de la viscosité ce qui ne permet plus dès lors d'obtenir une bonne diffusion.

Le second est le temps de séchage qui est particulièrement long par rapport aux compositions capillaires en solution hydroalcoolique ou alcoolique, ceci étant dû au fait que le véhicule est essentiellement de l'eau et que plus sa proportion est importante plus le temps de séchage sera long.

Après de nombreuses études sur un très grand nombre de polymères filmogènes capillaires, on vient maintenant de constater qu'il était possible d'apporter une solution à ces inconvénients et ainsi obtenir d'excellentes compositions cosmétiques aqueuses pour la fixation de la chevelure en utilisant une dispersion aqueuse de particules d'un polymère filmogène et au moins un agent plastifiant.

Les dispersions aqueuses de particules d'un polymère filmogène sont généralement désignées par les expressions "latex" ou "pseudo-latex".

Toutefois l'expression "pseudo-latex"désigne une dispersion constituée de particules généralement sphériques, d'un polymère filmogène, celles-ci étant obtenues par dispersion du polymère dans une phase aqueuse appropriée.

Cette expression ne doit pas être confondue avec l'expression "latex" ou "latex synthétique" qui est également une dispersion constituée de particules d'un polymère qui sont obtenues directement par polymérisation d'un ou plusieurs monomères dans une phase aqueuse appropriée.

Selon l'invention la dispersion aqueuse de particules de polymère filmogène est du type pseudo-latex" et dans la mesure où celle-ci contient un agent plastifiant, celle-ci sera désignée par la suite sous l'expression"pseudo-latex plastifié".

L'emploi d'un pseudo-latex plastifié permet d'apporter aux compositions cosmétiques pour la fixation de la chevelure de très nombreux avantages, et en particulier :
1. un temps de séchage beaucoup plus rapide, ceci étant dû au fait que l'on peut utiliser moins d'eau tout en ayant une concentration élevée en polymère filmogène sans que l'on rencontre des problèmes de viscosité, le polymère étant en dispersion dans la phase aqueuse et non en solution,
2. la possibilité d'utiliser dans les laques aérosols une quantité élevée d'agent propulseur tel que le diméthyléther avec lequel aucune démixtion n'a été observée avec des quantités supérieures à 35% en poids, ce qui favorise également un meilleur temps de séchage,
3. l'obtention d'excellentes propriétés cosmétiques concernant le pouvoir fixant, le démêlage, l'aspect des cheveux au toucher et l'absence de poudrage,
4. une bonne élimination au shampooing sans qu'il soit nécessaire d'utiliser un shampooing spécifique,
5. une absence d'aspect blanchâtre des cheveux avant séchage notamment lorsque l'agent propulseur des laques aérosols est le diméthyléther, ceci étant dû au fait que la taille des particules est très petite.

La présente invention a pour objet à titre de produit industriel nouveau, une composition cosmétique aqueuse pour la fixation de la chevelure, celle-ci étant constituée par une dispersion aqueuse :
a) de particules d'un polymère fllmogène à fonctions acides carboxyliques ayant un diamètre moyen compris entre 10 et 300nm, ledit polymère étant choisi parmi :
   (i) les copolymères acétate de vinyle/acide crotonique polyoxyéthylénés,
   (ii) les copolymères acétate de vinyle/acide crotonique,
   (iii) les terpolymères acétate de vinyle/acide crotonique/néodécanoate de vinyle,
   (iv) les copolymères N-octylacrylamide/méthacrylate de méthyle/méthacrylate d'hydroxypropyle/acide acrylique/méthacrylate de tert-butylaminoéthyle,
   (v) les copolymères alternés méthylvinyléther/anhydride maléique monoestérifiés par le butanol,
   (vi) les terpolymères acide acrylique/acrylate d'éthyle/N-tert-butylacrylamide, et
   (vii) les polymères répondant à la formule générale suivante : 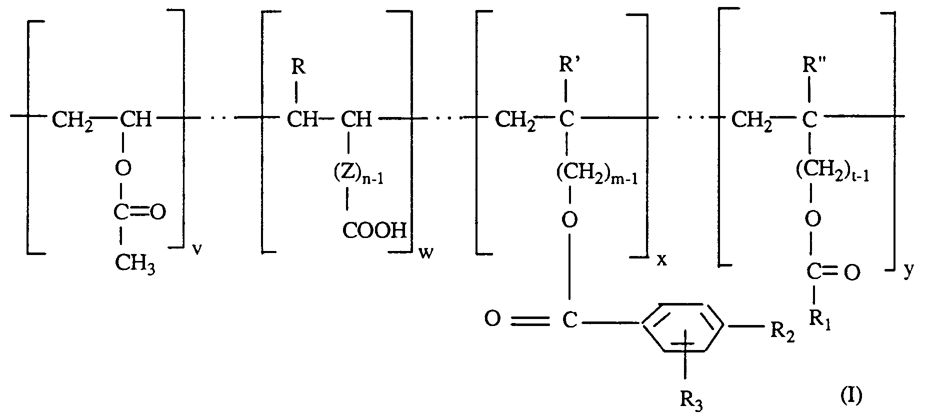 dans laquelle :
      R, R' et R'', identiques ou différents, représentent un atome d'hydrogène ou un radical méthyle,
      m, n et t sont 1 ou 2,
      R₁ représente un radical alkyle, linéaire ou ramifié, saturé ou insaturé ayant de 2 à 21 atomes de carbone,
      R₂ représente un atome d'hydrogène, un radical méthyle, éthyle, tert-butyle, éthoxy, butoxy ou dodécyloxy,
      R₃ représente un atome d'hydrogène, un radical alkyle de 1 à 4 atomes de carbone ou un radical alkoxy de 1 à 4 atomes de carbone,
      Z représente un radical divalent pris dans le groupe constitué par : -CH₂-, -CH₂-O-CH₂- et -CH₂-O-(CH₂)₂-,
      v représente de 10 à 91% et de préférence de 36 à 84% en poids,
      w représente de 3 à 20% et de préférence de 6 à 12% en poids,
      x représente de 4 à 60% et de préférence de 6 à 40% en poids, et
      y représente de 0 à 40% et de préférence de 4 à 30% en poids,
      v + w + x + y étant égal à 100%, et
      les fonctions acides carboxyliques dudit polymère étant neutralisées à un taux de neutralisation compris entre 10 et 80% à l'aide d'un agent monobasique non volatil, et
(b) d'au moins un agent plastifiant, ledit agent étant distribué selon son coefficient de partage entre lesdites particules et la phase aqueuse.

Parmi les copolymères acétate de vinyle/acide crotonique polyoxyéthylénés, on peut notamment citer l'"Aristoflex A" d'indice d'acide 56 de la Société Hoechst.

Comme copolymères acétate de vinyle/acide crotonique, on peut citer le "Luviset CA66" d'indice d'acide 74 (acétate de vinyle 90/acide crotonique 10) de la Société BASF.

Parmi les terpolymères acétate de vinyle/acide crotonique/néodécanoate de vinyle, on peut citer la "Résine 28-29-30" d'indice d'acide 65 de la Société National Starch.

Comme copolymères N-octylacrylamide/méthacrylate de méthyle/méthacrylate d'hydroxypropyle/acide acrylique/méthacrylate de tert-butylaminoéthyle, on peut citer l'"Amphomer" d'indice d'acide 137 de la Société National Starch.

Parmi les copolymères alternés méthylvinyléther/anhydride maléique monoestérifiés par le butanol, on peut mentionner le "Gantrez ES425" d'indice d'acide 260 (méthylvinyléther 50/anhydride maléique 50) de la Société GAF.

Comme terpolymères acide acrylique/acrylate d'éthyle/N-tert-butylacrylamide, on peut citer en particulier l'"Ultrahold 8" d'indice d'acide 62 de la Société BASF.

Comme copolymères de formule (I), on peut citer ceux décrits dans le brevet français n°78.30596 (2.439.798) et en particulier les copolymères suivants :
. acétate de vinyle 65/acide crotonique 10/tert-butyl-4-benzoate de vinyle 25,
. acétate de vinyle 57/acide crotonique 10/tert-butyl-4-benzoate de vinyle 25/néodécanoate de vinyle 8,
. acétate de vinyle 70/acide crotonique 10/tert-butyl-4-benzoate de vinyle 10/néodécanoate de vinyle 10,
. acétate de vinyle 70/acide crotonique 10/benzoate de vinyle 10/néodécanoate de vinyle 10,
. acétate de vinyle 70/acide crotonique 10/tert-butyl-4-benzoate de vinyle10/stéarate d'allyle 10.

Selon l'invention, il est souhaitable que ladite dispersion aqueuse conduise après séchage à la formation d'un film dont l'énergie de surface est comprise entre 30 et 55 mJ/m² et la polarité entre 0 et 0,34 et de préférence inférieure à 0,25.

L'énergie de surface du film est déterminée en déposant une goutte d'eau et une goutte de diiodométhane sur le film, en mesurant l'angle formé par chaque goutte avec le film, puis en calculant l'énergie de surface dudit film d'après la méthode décrite dans "Wettability of Some Low Energy Surfaces", A.EL. Shimi, E.D. Goddard-Journal of Colloïd and Interface Science, Vol.48, n°2 (1974).

La polarité du film est calculée selon la méthode décrite dans "Polar and Nonpolar Interactions in Adhesion" S. Wu, J. Adhesion, vol.5, pp39-55, (1973).

Les polymères filmogènes à fonctions acides carboxyliques tel que définis ci-dessus sont des polymères synthétiques ayant de préférence un poids moléculaire moyen compris entre 5 000 et 700 000 mesuré par exemple en chromatographie d'exclusion stérique.

Ces polymères sont insolubles dans l'eau et sont pour l'essentiel des polymères filmogènes couramment utilisés pour la réalisation de compositions cosmétiques pour la fixation de la chevelure.

Les pseudo-latex ou dispersions aqueuses de particules de polymère filmogène des compositions cosmétiques selon l'invention sont obtenus selon les méthodes connues de préparation des pseudo-latex sous réserve toutefois de certaines particularités qui seront mentionnées ci-après.

Le procédé général de préparation des pseudo-latex consiste à dissoudre un polymère insoluble dans l'eau dans un solvant organique, soluble ou partiellement soluble dans l'eau, à disperser sous agitation la solution ainsi obtenue dans de l'eau et à procéder ensuite à l'élimination du solvant organique par évaporation sous vide ce qui conduit à une suspension constituée de particules du polymère dont la taille est généralement inférieure au µm.

Selon ce procédé général, l'emploi d'un tensio-actif, d'un mélange de tensio-actifs ou d'un polymère colloïde protecteur ou encore d'un mélange tensio-actif/polymère colloïde protecteur est indispensable, en vue d'obtenir une bonne stabilisation des particules.

Les polymères filmogènes à fonctions acides carboxyliques tels que définis ci-dessus ne peuvent être utilisés tels quels dans la préparation des pseudo-latex mais doivent être neutralisés à un taux de neutralisation inférieur à 100% en vue d'éviter leur totale solubilisation dans l'eau.

Par une neutralisation partielle des polymères, on a constaté qu'il était possible d'obtenir des pseudo-latex particulièrement stables en l'absence de stabilisant hydrophile ou de tensio-actif ou encore de colloïde protecteur.

Le taux de neutralisation des polymères filmogènes à fonctions acides carboxyliques doit donc être parfaitement déterminé de telle sorte qu'ils restent insolubles dans l'eau tout en étant solubles dans le solvant organique.

Il va de soi que le taux limite supérieur de neutralisation qu'il conviendra de ne pas excéder pour que le polymère reste insoluble dans l'eau sera fonction de la nature de chaque polymère filmogène à fonctions acides carboxyliques. De façon générale, ce taux de neutralisation est généralement compris entre 30 et 80% et de préférence entre 40 et 70% si le polymère a moins de 2meq/g de fonctions acides carboxyliques et entre 10 et 50% de préférence entre 10 et 40% si le polymère a plus de 2meq/g de fonctions acides carboxyliques.

Selon l'invention la neutralisation des fonctions acides carboxyliques est réalisée à l'aide d'un agent mono-basique non volatil choisi par exemple parmi une base minérale telle que la soude ou la potasse ou parmi un aminoalcool pris dans le groupe constitué par l'amino-2 méthyl-2 propanol-1 (AMP), la triéthanolamine, la triisopropanolamine (TIPA), la monoéthanolamine, la diéthanolamine, la tri [(hydroxy-2) propyl-1] amine, l'amino-2 méthyl-2 propanediol-1,3 (AMPD) et l'amino-2 hydroxyméthyl-2 propanediol-1,3.

Dans la préparation des pseudo-latex, utilisés dans les compositions selon l'invention, la neutralisation des fonctions acides carboxyliques du polymère filmogène est réalisée in situ dans la solution du polymère dans le solvant organique par addition de la quantité déterminée du composé mono-basique non volatil. Le solvant organique utilisé doit être un solvant volatil ou un mélange de tels solvants présentant un point d'ébullition inférieur à celui de l'eau et être miscible ou partiellement miscible à l'eau.

Le solvant organique tel que défini ci-dessus est de préférence choisi parmi l'acétone, la méthyléthylcétone, le tétrahydrofuranne, l'acétate de méthyle, l'acétate d'éthyle, l'isopropanol et l'éthanol.

Après l'obtention de la solution du polymère partiellement neutralisé dans le solvant organique, on procède alors à la préparation d'une émulsion en versant sous agitation, à la solution organique obtenue, une quantité appropriée d'eau contenant éventuellement un agent anti-mousse dont le rôle sera de faciliter l'évaporation ultérieure de la phase organique.

Selon une variante du procédé tel que défini ci-dessus la neutralisation des fonctions acides carboxyliques du polymère en solution dans le solvant organique peut être réalisée lors de la formation de l'émulsion en versant une solution aqueuse contenant la quantité requise du composé mono-basique non volatil.

Lors de la formation de l'émulsion l'agitation est de préférence réalisée à l'aide d'un disperseur cisaillant du type Moritz ou Ultra-Turrax ou Raineri équipé de pales défloculantes.

L'émulsion ainsi obtenue est particulièrement stable sans qu'il soit nécessaire d'employer un agent tensio-actif dans la mesure où les groupes carboxylates du polymère se placent à l'interface avec l'eau et protègent les gouttelettes de la coalescence par répulsion électrostatique.

Après formation de l'émulsion à une température comprise entre la température ambiante et 70°C environ, on procède alors à l'évaporation sous pression réduite du solvant organique jusqu'à son élimination totale, l'évaporation étant de préférence réalisée sous léger chauffage.

On obtient ainsi un pseudo-latex, c'est-à-dire une dispersion aqueuse de particules du polymère filmogène, qui est exempt de tout tensio-actif ou autre stabilisant hydrophile tout en étant très stable ce qui est particulièrement avantageux en cosmétique capillaire. En effet la présence d'un tensio-actif dans une laque aérosol apporte très souvent de mauvaises propriétés cosmétiques sur cheveux et une sensibilité à l'hygroscopicité qui se traduit par un poissage au toucher désagréable.

La taille moyenne des particules est comprise entre 10 et 300nm mais est de préférence inférieure à 250nm.

La polydispersité en taille des particules est relativement faible selon ce procédé de préparation du pseudo-latex, celle-ci mesurée en diffusion quasi élastique de lumière est généralement comprise entre 0,1 et 0,40 et de préférence inférieure à 0,35.

Comme ceci est bien connu, les agents plastifiants sont généralement des composés liquides de point d'ébullition élevé qui permettent d'abaisser la température de transition vitreuse ainsi que la température de ramollissement des polymères leur conférant ainsi une meilleure flexibilité.

L'agent plastifiant est généralement présent en une proportion comprise entre 0,1 et 80%, de préférence comprise entre 5 et 40% et en particulier entre 10 et 30% en poids par rapport au poids du polymère filmogène neutralisé.

L'agent plastifiant qui peut être du type hydrophile ou hydrophobe, est de préférence introduit en mélange dans le solvant organique lors de la préparation du pseudo-latex, et notamment lorsqu'il est du type hydrophobe.

Lorsque l'agent plastifiant est du type hydrophile, il peut être introduit dans la dispersion après formation du pseudo-latex.

Parmi les agents plastifiants pouvant être utilisés selon l'invention, on peut citer :
- les Carbitols de la Société Union Carbide à savoir le Carbitol ou diéthylène glycol éthyléther, le méthyl Carbitol ou diéthylène glycol méthyléther, le butyl Carbitol ou diéthylène glycol buthyléther ou encore l'hexyl Carbitol ou diéthylène glycol hexyléther,
- les Cellosolves de la Société Union Carbide à savoir le Cellosolve ou éthylène glycol éthyléther, le butyl Cellosolve ou éthylène glycol butyléther, l'hexyl Cellosolve ou éthylène glycol hexyléther,
- les dérivés de propylène glycol et en particulier le propylène glycol phényléther, le propylène glycol diacétate, le dipropylène glycol butyléther, le tripropylène glycol butyléther, ainsi que les Dowanols de la Société Dow Chemical à savoir le Dowanol PM ou propylène glycol méthyléther, le Dowanol DPM ou dipropylène glycol méthyléther et le Dowanol TPM ou tripropylène glycol méthyléther.

On peut encore citer :
- le diéthylène glycol méthyléther ou Dowanol DM de la Société Dow Chemical,
- l'huile de ricin oxyéthylénée à 40 moles d'oxyde d'éthylène telle que celle vendue par la Société Rhône Poulenc sous la dénomination de "Mulgofen EL-719",
- l'alcool benzylique,
- le citrate de triéthyle vendu par la Société Pfizer sous la dénomination de "Citroflex-2",
- le 1,3-butylène glycol,
- les phtalates et adipates de diéthyle, de dibutyle et de diisopropyle,
- les tartrates de diéthyle et de dibutyle,
- les phosphates de diéthyle, de dibutyle et de diéthyl-2 hexyle, et
- les esters de glycérol tels que le diacétate de glycérol (diacétine) et le triacétate de glycérol (triacétine).

L'agent plastifiant est choisi de préférence dans le groupe constitué par le dipropylène glycol méthyléther, le tripropylène glycol méthyléther, l'adipate de diéthyle et l'adipate de diisopropyle.

La concentration en poids du polymère filmogène sous forme de particules dans le pseudo-latex obtenu par le procédé tel que décrit ci-dessus est généralement comprise entre 5 et 50% et de préférence entre 10 et 25% par rapport au poids total du pseudo-latex.

La viscosité des pseudo-latex à une concentration de 25% en poids de particules de polymère filmogène doit de préférence être comprise entre 10 cP et 3000 cP (mesurée au Contraves à 25°C).

Les compositions cosmétiques capillaires pour la fixation de la chevelure selon l'invention se présentent, comme mentionné ci-dessus, sous forme d'une laque aérosol, d'une lotion de fixation pour pulvérisation manuelle, d'une lotion de mise en plis ou encore d'une mousse de coiffage. La proportion en poids de polymère dans ces compositions est comprise entre 1 et 40% par rapport au poids total de la composition.

L'utilisation d'un pseudo-latex plastifié tel que défini ci-dessus dans la réalisation de laques aérosols présente de très nombreux avantages, par rapport aux laques aérosols usuelles dans lesquelles les mêmes polymères sont utilisés à l'état dissous en solution aqueuse, hydroalcoolique ou alcoolique.

Le principal avantage est de pouvoir obtenir des laques à l'eau présentant toutes les propriétés cosmétiques recherchées et un temps de séchage beaucoup plus rapide que les laques à l'eau usuelles du fait de la présence d'une quantité d'eau beaucoup plus faible.

Par ailleurs, ce temps de séchage est également favorisé dans certains cas par le fait qu'en utilisant un pseudo-latex plastifié on peut augmenter de façon particulièrement significative la proportion de l'agent propulseur par rapport à la phase aqueuse contenant le pseudo-latex sans qu'il y ait démixtion et apparition d'une deuxième phase liquide.

Ceci est tout particulièrement vrai et a pu être mis en évidence lorsque l'agent propulseur est du diméthyléther qui est un gaz liquéfié partiellement miscible à l'eau.

Avec cet agent propulseur il est ainsi possible d'obtenir d'excellentes laques aérosols en contenant une proportion supérieure ou égale à 40%.

Par contre lorsque le polymère filmogène est en solution dans la phase aqueuse et non plus sous forme de pseudo-latex, la proportion d'agent propulseur tel que le diméthyléther doit être maintenue à une teneur beaucoup plus faible si l'on veut éviter une séparation de phase. Il en résulte donc une phase aqueuse relativement importante généralement supérieure à 65% environ ce qui a pour conséquence un temps de séchage beaucoup plus long.

Bien qu'il ait été fait référence ci-dessus à l'emploi du diméthyléther en tant qu'agent propulseur, il va de soi que d'autres agents propulseurs peuvent être employés selon l'invention pour la réalisation des laques aérosols.

Parmi ceux-ci on peut notamment mentionner l'azote, l'air comprimé, l'anhydride carbonique, les hydrocarbures tels que le propane, le butane et l'isobutane, les dérivés fluorés des hydrocarbures aliphatiques et cycliques et notamment les difluoroéthanes, les tétrafluoroéthanes et l'octafluorocyclobutane, ceux-ci pouvant être utilisés seuls ou en mélange. On utilise de préférence selon l'invention le diméthyléther.

Dans les laques aérosols selon l'invention, on utilise le pseudo-latex plastifié en une proportion telle que le polymère filmogène dispersé soit présent à une concentration comprise entre 2 et 30% et de préférence entre 5 et 20% en poids par rapport au poids total de la composition.

La proportion totale en eau est généralement comprise entre 38 et 63% en poids et de préférence entre 45 et 60% par rapport au poids total de la composition.

La proportion en agent propulseur lorsqu'il est liquéfiable tel que le diméthyléther est généralement comprise entre 20 et 60%, de préférence entre 30 et 55% de façon à conduire au sein du récipient aérosol à une seule phase liquéfiée.

Lorsque la composition capillaire selon l'invention se présente sous forme d'une lotion de fixation ou de mise en plis, le pseudo-latex plastifié est généralement présent en une proportion telle que la composition présente une teneur en polymère filmogène comprise entre 2 et 30% et de préférence entre 5 et 20%, la teneur en eau totale étant comprise entre 70 et 86% en poids par rapport au poids total de la composition.

Lorsque la composition selon l'invention se présente sous forme d'une mousse de coiffage, le pseudo-latex plastifié est généralement présent en une proportion telle que la composition présente une teneur en polymère filmogène comprise entre 2 et 15% et de préférence entre 2 et 10%. La teneur en eau est de préférence comprise entre 70 et 90% et celle de l'agent propulseur entre 2 et 15% en poids, l'agent propulseur pouvant être l'un de ceux tels que mentionnés ci-dessus.

Les compositions cosmétiques pour la fixation de la chevelure selon l'invention peuvent également contenir divers additifs cosmétiques tels que des filtres solaires, des polymères dont l'homopolymère réticulé du chlorure de méthacryloyloxyéthyltriméthylammonium et les copolymères réticulés de l'acrylamide et d'un monomère choisi parmi l'acide 2-acrylamide 2-méthyl propane sulfonique neutralisé, l'acrylate d'ammonium et le chlorure de méthacryloyloxyéthyltriméthylammonium, des protéines, des silicones organo-modifiées ou non, volatiles ou non, des agents anti-mousses, des agents hydratants, des humectants, des parfums, des conservateurs, des colorants, des anti-oxydants, etc....

On va maintenant donner à titre d'illustration plusieurs exemples de préparation de pseudo-latex plastifié.

### EXEMPLES DE PREPARATION DES PSEUDO-LATEX

### EXEMPLE 1 : Préparation du pseudo-latex plastifié copolymère acétate de vinyle/acide crotonique polyoxyéthyléné ("Aristoflex A" de la Société Hoechst)-monométhyléther de tripropylène glycol.

50g du copolymère "Aristoflex A" sous forme de poudre (indice d'acide mesuré = 56) sont additionnés petit à petit sous agitation à une solution homogène de 186,9g de méthyléthylcétone, 3,115g d'amino-2 méthyl-2 propanol-1 (AMP) (quantité correspondante à 70% de neutralisation d'après l'indice d'acide du copolymère "Aristoflex A") et 10g de monométhyléther de tripropylène glycol.

Après poursuite de l'agitation pendant 30 minutes, la dissolution du copolymère est totale.

A la phase organique ainsi obtenue on ajoute sous agitation à l'aide d'un disperseur Moritz à 2500tr/min une phase aqueuse, pour réaliser l'émulsion, celle-ci étant constituée par 274,45 g d'eau permutée et 0,57g d'un agent anti-mousse siliconé "Burst RSD10".

L'addition de la phase aqueuse est réalisée en environ 15 minutes puis on maintient l'agitation à 3000tr/min pendant 15 minutes. On obtient ainsi une émulsion d'aspect laiteux et moyennement visqueuse.

On procède alors à l'évaporation de tout le solvant organique à l'aide d'un évaporateur rotatif sous vide partiel et chauffage à environ 40-45°C. L'évaporation est poursuivie jusqu'à élimination totale de la méthyléthylcétone en respectant l'azéotrope avec l'eau.

La quantité d'eau éliminée par formation de l'azéotrope est ensuite rajoutée à la dispersion pour obtenir un pseudo-latex plastifié ayant une concentration en polymère filmogène de 15% et en agent plastifiant de 3%. Le pseudo-latex plastifié obtenu est stable, d'un aspect opaque légèrement bleuté et moyennement visqueux.

La taille des particules a été mesurée en diffusion quasi élastique de lumière au Coulter modèle M4 et a donné les résultats suivants :
- taille moyenne des particules : 207nm
- facteur de polydispersité = 0,11

Selon le même mode opératoire tel que décrit ci-dessus on a également préparé des pseudo-latex plastifiés à l'aide des copolymères commerciaux "Luviset CA 66", "Amphomer LV 71", "Gantrez ES425" et "Résine 28-29-30" contenant comme agent plastifiant du monométhyléther de tripropylène glycol ou de l'adipate de diisopropyle.

Les conditions d'obtention et caractéristiques des pseudo-latex plastifiés obtenus sont données dans le tableau I ci-après.

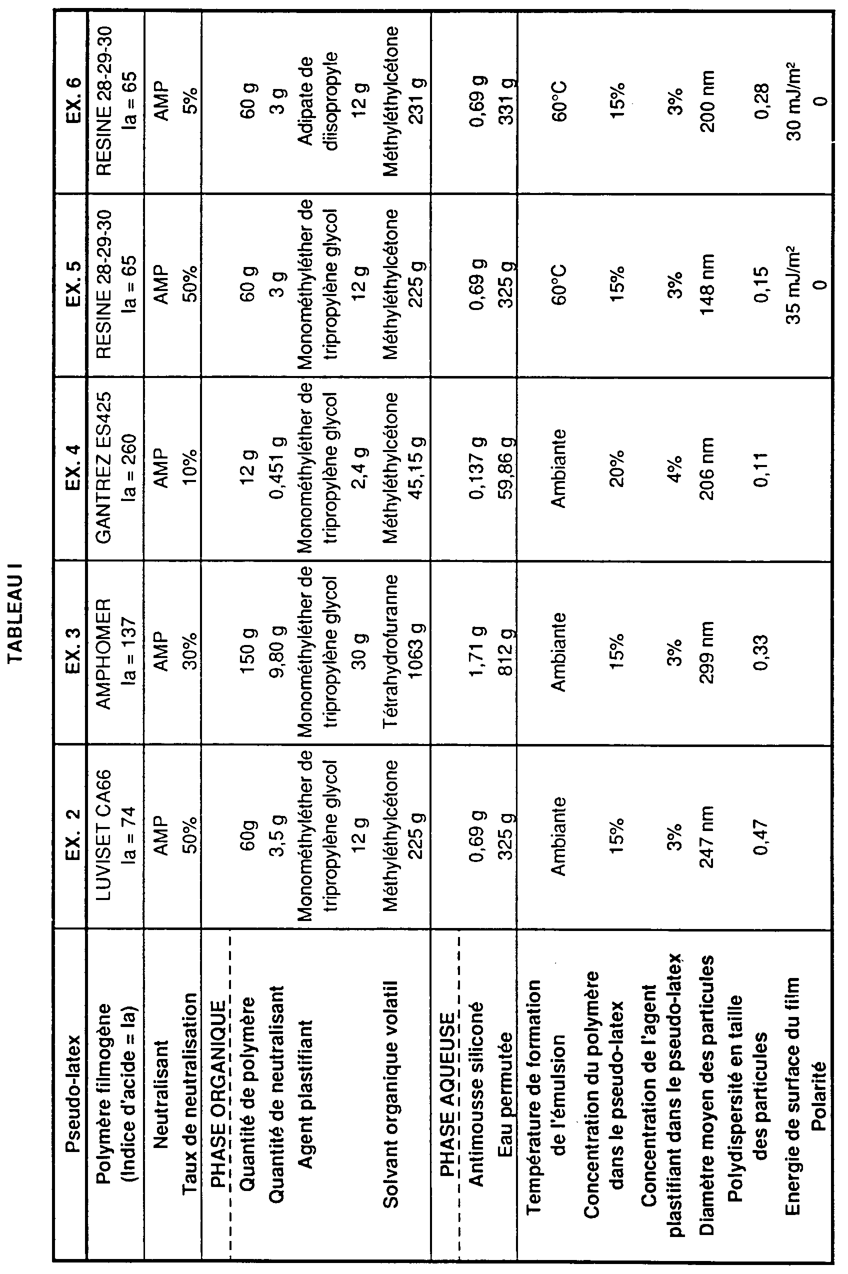

### EXEMPLE 7 : Préparation du pseudo-latex plastifié copolymère acétate de vinyle/acide crotonique/tert-butyl-4-benzoate de vinyle (65/10/25)-adipate de diéthyle.

La préparation de ce copolymère est décrite à l'exemple 19 du brevet français n° 78.30596 (n° 2.439.798) et se présente sous forme de billes ayant un diamètre de 0,5 à 1 mm.

100g du copolymère défini ci-dessus (Indice d'acide : 65) sont additionnés petit à petit sous agitation à une solution homogène de 280g d'acétone, 6,2g d'amino-2 méthyl-2 propanol-1 (quantité correspondante à 50% de neutralisation d'après l'indice d'acide) et 20g d'adipate de diéthyle.

Après agitation à température ambiante pendant 30 minutes, la dissolution du polymère est totale.

A la phase organique ainsi obtenue on ajoute en 5 minutes environ sous agitation à l'aide d'un disperseur cisaillant du type Ultra-Turrax à 2000tr/min, une phase aqueuse pour réaliser l'émulsion, celle-ci étant constituée de 265,55g d'eau permutée et 1,14g d'un agent anti-mousse siliconé "Burst RSD 10".

Après la fin de l'addition de la phase aqueuse, à température ambiante, on poursuit l'agitation pendant 10 à 15 min, ce qui permet de conduire à l'obtention d'une émulsion translucide et stable.

On procède alors à la concentration à l'aide d'un évaporateur rotatif sous vide partiel à une température inférieure à 45°C. Après élimination totale de l'acétone on obtient une dispersion stable laiteuse et peu visqueuse.

La concentration en polymère filmogène est de 25% et celle en agent plastifiant de 5%.

La taille des particules a été mesurée en diffusion quasi élastique de lumière au Coulter modèle M4 et a donné les résultats suivants :
Taille moyenne des particules : 161nm
Facteur de polydispersité : 0,25

Les caractéristiques du film obtenu après séchage du pseudo-latex plastifié sont :
- Energie de surface : 42 mJ/m²
- Polarité : 0,29

Selon le même mode opératoire tel que décrit ci-dessus on a également préparé d'autres pseudo-latex plastifiés à partir du même polymère de l'exemple 19 du brevet français n° 78.30596 (n° 2.439.798) mais en faisant varier le taux de neutralisation et en employant d'autres agents neutralisants.

Les caractéristiques des pseudo-latex plastifiés obtenus ainsi que les conditions d'obtention sont données dans le tableau II ci-après.

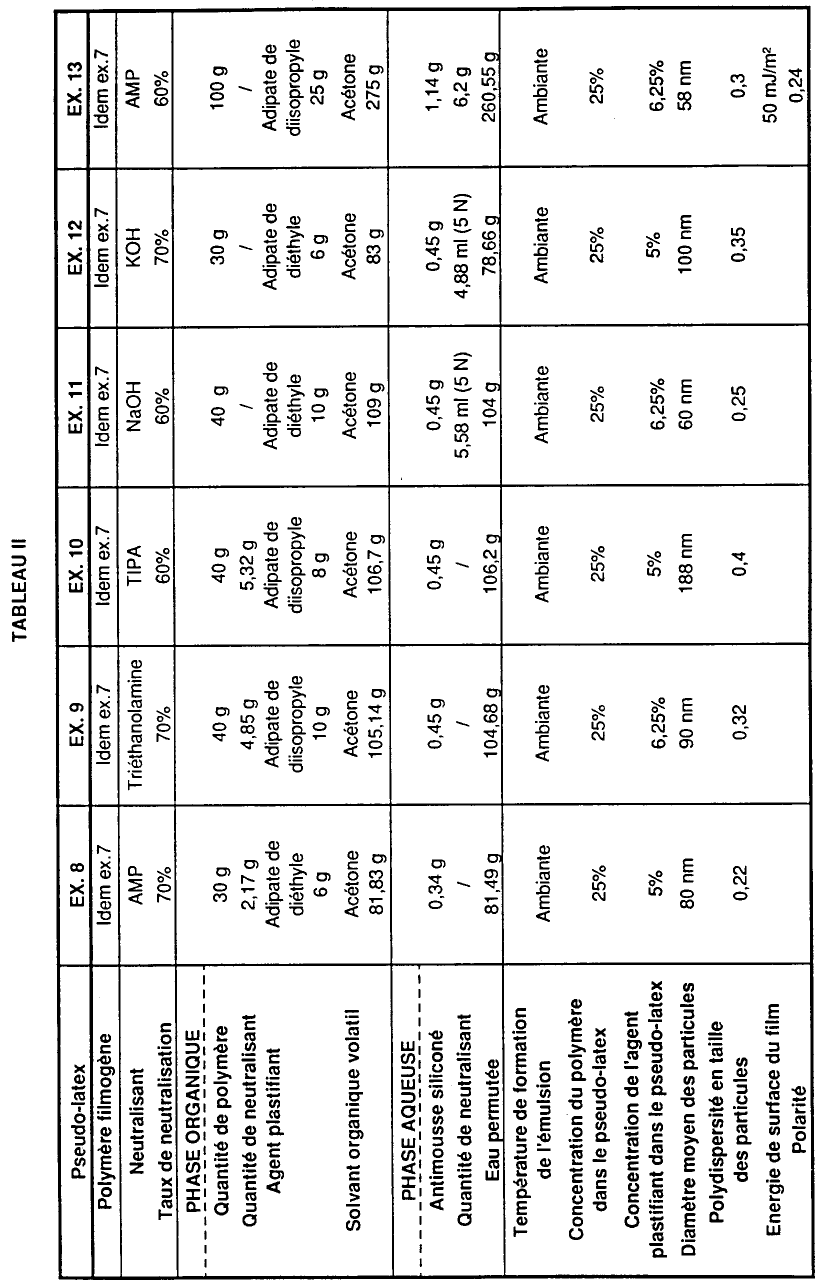

On a également préparé différents pseudo-latex plastifiés à partir d'autres polymères filmogènes décrits dans le brevet français n° 78 30596.

Les polymères utilisés ont été les suivants:

### Polymère A

Polymère acétate de vinyle/acide crotonique/tert-butyl-4 benzoate de vinyle/néodécanoate de vinyle (57/10/25/8).

### Polymère B

Polymère acétate de vinyle/acide crotonique/tert-butyl-4 benzoate de vinyle/néodécanoate de vinyle (70/10/10/10).

### Polymère C

Polymère acétate de vinyle/acide crotonique/benzoate de vinyle/néodécanoate de vinyle (70/10/10/10).

### Polymère D

Polymère acétate de vinyle/acide crotonique/tert-butyl-4 benzoate de vinyle/stéarate d'allyle (70/10/10/10).

A partir des polymères filmogènes A à D, on a préparé des pseudo-latex plastitifés selon le même mode opératoire que celui décrit à l'exemple 7. On donne ci-après dans le tableau III les conditions d'obtention et caractéristiques des pseudo-latex plastifiés obtenus.

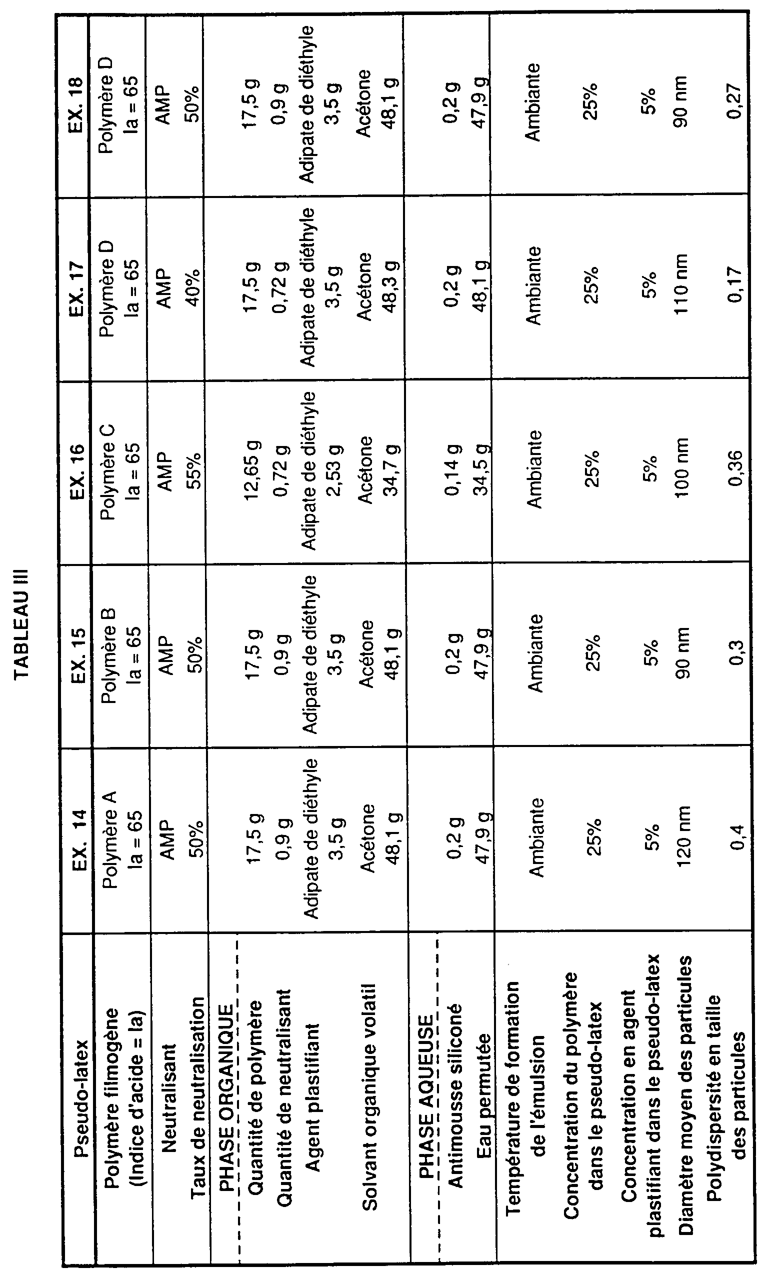

### EXEMPLE 19 : Préparation du pseudo-latex plastifié terpolymère acétate de vinyle/acide crotonique/néodécanoate de vinyle ("Résine 28-29-30" de la Société National Starch)-monométhyléther de tripropylène glycol.

60g de terpolymère "Résine 28-29-30" sous forme de poudre sont additionnés sous agitation à une solution de 225g de méthyléthylcétone et de 3g d'amino-2-méthyl-2 propanol-1 (quantité correspondante à 50% de neutralisation d'après l'indice d'acide du terpolymère "Résine 28-29-30")

Après poursuite de l'agitation pendant 30 minutes, la dissolution du copolymère est totale.

A la phase organique ainsi obtenue on ajoute sous agitation à l'aide d'un disperseur Moritz à 2500tr/min une phase aqueuse, pour réaliser l'émulsion, celle-ci étant constituée par 325g d'eau permutée et 0,69g d'agent anti-mousse siliconé "Burst RSD10".

L'addition de la phase aqueuse est réalisée en environ 15 minutes puis on maintient l'agitation à 3000tr/min pendant 15 minutes. On obtient ainsi une émulsion d'aspect laiteux et moyennement visqueuse.

On procède ensuite à l'évaporation de tout le solvant organique à l'aide d'un évaporateur rotatif sous vide partiel et chauffage à environ 40-45°C. L'évaporation est poursuivie jusqu'à élimination totale de la méthyléthylcétone en respectant l'azéotrope avec l'eau.

La quantité d'eau éliminée par formation de l'azéotrope est ensuite rajoutée à la dispersion pour obtenir un pseudo-latex ayant une concentration en polymère filmogène de 15,4%.

A la dispersion obtenue, on ajoute, sous agitation, 12g de monométhyléther de tripropylène glycol, quantité correspondant à 20% en poids de plastifiant par rapport au poids de polymère non neutralisé. On laisse agiter pendant 48 heures à température ambiante. Le pseudo-latex plastifié ainsi obtenu est stable et contient 15% de polymère et 3% de plastifiant.

La taille des particules a été mesurée en diffusion quasi élastique de lumière au Coulter modèle M4 et a donné les résultats suivants :
Taille moyenne des particules : 140 nm
Facteur de polydispersité : 0,20

Les caractéristiques de ce pseudo-latex plastifié sont très similaires à celles du pseudo-latex plastifié obtenu selon le procédé de l'exemple 5 dans lequel l'agent plastifiant est introduit en mélange dans le solvant organique.

### EXEMPLES DE FORMULATION

### EXEMPLE A :

On prépare une laque aérosol pour cheveux en conditionnant, dans un récipient aérosol approprié :

| | |
|---|---|
| - Pseudo-latex plastifié de l'Exemple 7 | 52g |
| - Diméthyléther | 45g |
| - Parfum | 0,05g |
| - Eau q.s.p. | 100g |

Le mélange ne constitue qu'une seule phase.

On procède à la fixation de la valve et à la fermeture hermétique du récipient.

La laque appliquée sur la chevelure ne présente pas d'aspect blanchâtre, sèche rapidement, possède un bon pouvoir laquant, une bonne tenue et ne provoque pas d'effet de poissage lors de l'application et après séchage.

Le film de polymère s'élimine facilement au shampooing.

### EXEMPLE B :

Selon le même mode opératoire que décrit à l'Exemple A, on a préparé une laque aérosol pour cheveux ayant la composition suivante :

| | |
|---|---|
| - Pseudo-latex plastifié de l'Exemple 10 | 36g |
| - Diméthyléther | 48g |
| - Parfum | 0,05g |
| - Eau q.s.p. | 100g |

Le mélange se présente sous forme d'une seule phase homogène.

Après pulvérisation le séchage est rapide et l'on obtient une excellente tenue des cheveux.

La laque s'élimine facilement au shampooing.

### EXEMPLE C :

Selon le même mode opératoire que décrit à l'Exemple A, on a préparé une laque aérosol pour cheveux ayant la composition suivante :

| | |
|---|---|
| - Pseudo-latex plastifié de l'Exemple 18 | 40g |
| - Parfum, colorant, conservateur q.s. | |
| - Diméthyléther | 50g |
| - Eau q.s.p. | 100g |

Le mélange se présente sous forme d'une seule phase homogène.

### EXEMPLE D :

Selon le même mode opératoire que décrit à l'Exemple A, on a préparé une laque aérosol pour cheveux ayant la composition suivante :

| | |
|---|---|
| - Pseudo-latex plastifié de l'Exemple 14 | 24g |
| - Parfum, colorant, conservateur q.s. | |
| - Diméthyléther | 30g |
| - Eau q.s.p. | 100g |

### EXEMPLE E :

On prépare un spray de fixation en flacon pompe en conditionnant, dans un récipient approprié :

| | |
|---|---|
| - Pseudo-latex plastifié de l'Exemple 16 | 32g |
| - Parfum, colorant, conservateur q.s. | |
| - Eau q.s.p. | 100g |

Le récipient, une fois rempli, est ensuite équipé d'une pompe de pulvérisation.

### EXEMPLE F :

On prépare un spray de fixation en procédant au mélange suivant :

| | |
|---|---|
| - Pseudo-latex plastifié de l'Exemple 15 | 60g |
| - Parfum, colorant, conservateur q.s. | |
| - Eau q.s.p. | 100g |

La lotion obtenue est ensuite conditionnée dans un pulvérisateur rechargeable en air comprimé.

### EXEMPLE G :

On prépare une lotion de mise en plis ayant la composition suivante :

| | |
|---|---|
| - Pseudo-latex plastifié de l'Exemple 15 | 40g |
| - Parfum, colorant, conservateur q.s. | |
| - Eau q.s.p. | 100g |

### EXEMPLE H :

On prépare une mousse de coiffage aérosol ayant la composition suivante :

| | |
|---|---|
| - Pseudo-latex plastifié de l'Exemple 7 | 4g |
| - Alcool polyvinylique vendu sous la dénomination de "Mowiol 4088" par la Société Hoechst | 0,5g |
| - Parfum, colorant, conservateur q.s. | |
| - Eau q.s.p. | 100g |

On introduit 90g de la composition obtenue dans un boîtier aérosol sans tube plongeur. On procède à la fixation de la valve et à la fermeture hermétique du récipient, puis on introduit 10g d'un mélange propulseur butane/iso-butane/propane (3,2 bars).

### EXEMPLE I :

Selon le même mode opératoire qu'à l'Exemple A, on a préparé une laque aérosol pour cheveux ayant la composition suivante :

| | |
|---|---|
| - Pseudo-latex plastifié de l'Exemple 15 | 28g |
| - Parfum, colorant, conservateur q.s. | |
| - Diméthyléther | 30g |
| - Eau q.s.p. | 100g |

Le mélange se présente sous forme d'une seule phase homogène.

La laque appliquée sur la chevelure présente un faible temps de séchage et un bon pouvoir laquant.

Le film s'élimine facilement au brossage et au shampooing.

### EXEMPLE J :

Selon le même mode opératoire que décrit à l'Exemple H, on a préparé une mousse de coiffage ayant la composition suivante :

| | |
|---|---|
| - Pseudo-latex plastifié de l'Exemple 7 | 40g |
| - Ester cétylique d'hydroxyéthylcellulose vendu sous la dénomination de "Natrosol Plus 330 CS" par la Société Aqualon | 0,5g |
| - Parfum, colorant, conservateur q.s. | |
| - Eau déminéralisée q.s.p. | 100g |

On introduit 90g de la composition ainsi obtenue dans une bombe aérosol sans tube plongeur. On procède à la fixation de la valve et à la fermeture hermétique du récipient. On introduit ensuite 10g d'un mélange propulseur butane/isobutane/propane (3,2 bars).

Par pulvérisation on obtient une mousse rigide et ferme qui apporte un pouvoir de fixation ainsi que de bonnes propriétés cosmétiques notamment une bonne brillance.

### EXEMPLE K :

Selon le même mode opératoire que décrit à l'Exemple A, on a préparé une laque aérosol pour cheveux ayant la composition suivante :

| | |
|---|---|
| - Pseudo-latex plastifié de l'Exemple 5 | 59,4g |
| - Diméthyléther | 40g |
| - Parfum, colorant, conservateur q.s. | |
| - Eau déminéralisée q.s.p. | 100g |

### EXEMPLE L :

Selon le même mode opératoire que décrit à l'Exemple A, on a préparé une laque aérosol pour cheveux ayant la composition suivante :

| | |
|---|---|
| - Pseudo-latex plastifié de l'Exemple 4 | 25g |
| - Diméthyléther | 30g |
| - Parfum, colorant, conservateur q.s. | |
| - Eau déminéralisée q.s.p. | 100g |

### EXEMPLE M :

Selon le même mode opératoire que décrit à l'Exemple E, on a préparé un spray de fixation en flacon pompe ayant la composition suivante :

| | |
|---|---|
| - Pseudo-latex plastifié de l'Exemple 1 | 33,3g |
| - Parfum, colorant, conservateur q.s. | |
| - Eau déminéralisée q.s.p. | 100g |

### EXEMPLE N :

Selon le même mode opératoire que décrit à l'Exemple F, on a préparé un spray de fixation, conditionné dans un pulvérisateur rechargeable en air comprimé ayant la composition suivante :

| | |
|---|---|
| - Pseudo-latex plastifié de l'Exemple 2 | 46,7g |
| - Parfum, colorant, conservateur q.s. | |
| - Eau déminéralisée q.s.p. | 100g |

### EXEMPLE O :

On prépare une lotion de mise en plis ayant la composition suivante :

| | |
|---|---|
| - Pseudo-latex plastifié de l'Exemple 4 | 20g |
| - Parfum, colorant, conservateur q.s. | |
| - Eau déminéralisée q.s.p. | 100g |

### EXEMPLE P :

On prépare une mousse de coiffage aérosol ayant la composition suivante :

| | |
|---|---|
| - Pseudo-latex plastifié de l'Exemple 4 | 15g |
| - Alcool polyvinylique vendu sous la dénomination de "Mowiol 4088" par la Société Hoechst | 0,5g |
| - Parfum, colorant, conservateur q.s. | |
| - Eau q.s.p. | 100g |

On introduit 90g de la composition obtenue dans un boitier aérosol sans tube plongeur. On procède à la fixation de la valve et à la fermeture hermétique du récipient, puis on introduit 10g d'un mélange propulseur butane/propane/isobutane (3,2 bars).

### EXEMPLE Q :

Selon le même mode opératoire qu'à l'exemple A, on a préparé une laque aérosol pour cheveux ayant la composition suivante :

| | |
|---|---|
| - Pseudo-latex plastifié de l'Exemple 15 | 32g |
| - Parfum, colorant, conservateur q.s. | |
| - Diméthyléther | 40g |
| - Eau q.s.p. | 100g |

Le mélange se présente sous forme d'une seule phase homogène.

### EXEMPLE R :

Selon le même mode opératoire qu'à l'exemple A, on a préparé une laque aérosol pour cheveux ayant la composition suivante :

| | |
|---|---|
| - Pseudo-latex plastifié de l'Exemple 7 | 35,6g |
| - Diméthicone copolyol vendu sous la dénomination de "Silbione Huile 70646" par la Société Rhone Poulenc | 0,5g |
| - Parfum q.s. | |
| - Diméthyléther | 40g |
| - Eau q.s.p. | 100g |

Le mélange se présente sous forme d'une seule phase. On obtient une bonne pulvérisation, de bonnes qualités fixantes, de bonnes qualités cosmétiques, un bon démêlage au brossage et une bonne élimination au shampooing.

## Revendications

1. Composition cosmétique aqueuse pour la fixation de la chevelure caractérisée par le fait qu'elle est constituée par une dispersion aqueuse :
a) de particules d'un polymère filmogène à fonctions acides carboxyliques ayant un diamètre moyen compris entre 10 et 300nm, ledit polymère étant choisi parmi :
(i) les copolymères acétate de vinyle/acide crotonique polyoxyéthylénés,
(ii) les copolymères acétate de vinyle/acide crotonique,
(iii) les terpolymères acétate de vinyle/acide crotonique/néodécanoate de vinyle,
(iv) les copolymères N-octylacrylamide/méthacrylate de méthyle/méthacrylate d'hydroxypropyle/acide acrylique/méthacrylate de tert-butylaminoéthyle,
(v) les copolymères alternés méthylvinyléther/anhydride maléique monoestérifiés par le butanol,
(vi) les terpolymères acide acrylique/acrylate d'éthyle/N-tert-butylacrylamide, et
(vii) les polymères répondant à la formule générale suivante : 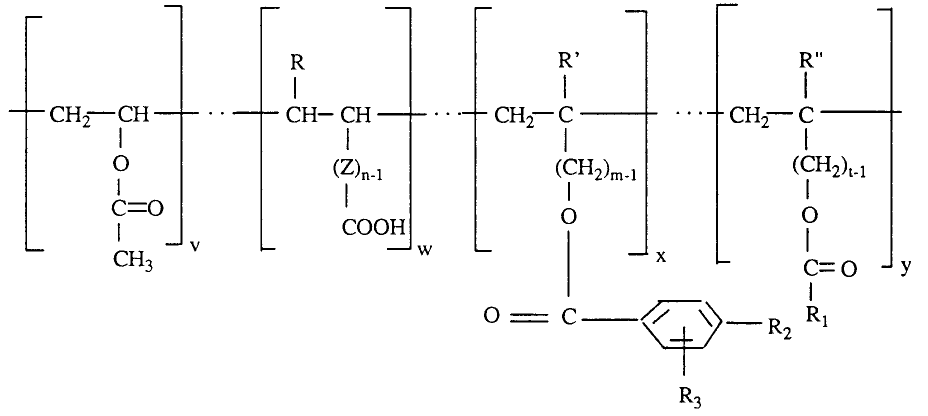 dans laquelle :
R, R' et R'', identiques ou différents, représentent un atome d'hydrogène ou un radical méthyle,
m, n et t sont 1 ou 2,
R₁ représente un radical alkyle, linéaire ou ramifié, saturé ou insaturé ayant de 2 à 2! atomes de carbone,
R₂ représente un atome d'hydrogène, un radical méthyle, éthyle, tert-butyle, éthoxy, butoxy ou dodécyloxy,
R₃ représente un atome d'hydrogène, un radical alkyle de 1 à 4 atomes de carbone ou un radical alkoxy de 1 à 4 atomes de carbone,
Z représente un radical divalent pris dans le groupe constitué par : -CH₂-, -CH₂-O-CH₂- et -CH₂-O-(CH₂)₂-,
v représente de 10 à 91% et de préférence de 36 à 84% en poids,
w représente de 3 à 20% et de préférence de 6 à 12% en poids,
x représente de 4 à 60% et de préférence de 6 à 40% en poids, et
y représente de 0 à 40% et de préférence de 4 à 30% en poids,
v + w + x + y étant égal à 100%,
les fonctions acides carboxyliques dudit polymère étant neutralisées à un taux de neutralisation compris entre 10 et 80% à l'aide d'un agent monobasique non volatil, et
b) d'au moins un agent plastifiant, ledit agent étant distribué selon son coefficient de partage entre lesdites particules et la phase aqueuse.

2. Composition cosmétique selon la revendication 1 caractérisée par le fait que ladite dispersion aqueuse conduit après séchage à la formation d'un film dont l'énergie de surface est comprise entre 30 et 55mJ/m² et la polarité entre 0 et 0,34 et de préférence inférieure à 0,25.

3. Composition cosmétique selon la revendication 1 ou 2, caractérisée par le fait que le polymère filmogène à fonctions acides carboxyliques a un poids moléculaire moyen compris entre 5 000 et 700 000.

4. Composition cosmétique selon l'une quelconque des revendications précédentes caractérisée par le fait que le polymère filmogène à fonctions acides carboxyliques est neutralisé à l'aide d'un agent mono-basique non volatil choisi parmi : la soude, la potasse, l'amino-2 méthyl-2 propanol, la triéthanolamine, la triisopropanolamine, la monoéthanolamine, la diéthanolamine, la tri[(hydroxy-2) propyl-1] amine, l'amino-2 méthyl-2 propanediol-1,3 et l'amino-2 hydroxyméthyl-2 propanediol-1,3.

5. Composition cosmétique selon l'une quelconque des revendications précédentes, caractérisée par le fait que si les fonctions acides carboxyliques du polymère filmogène ont moins de 2meq/g de fonctions acides carboxyliques, le taux de neutralisation est compris entre 30 et 80% et de préférence entre 40 et 70%.

6. Composition cosmétique selon l'une quelconque des revendications 1 à 4, caractérisée par le fait que si les fonctions acides carboxyliques, du polymère filmogène ont plus de 2meq/g de fonctions acides carboxyliques le taux de neutralisation est compris entre 10 et 50% et de préférence entre 10 et 40%.

7. Composition cosmétique selon l'une quelconque des revendications précédentes, caractérisée par le fait qu'elle contient de 1 à 40% en poids de particules du polymère filmogène par rapport au poids total de la composition.

8. Composition cosmétique selon l'une quelconque des revendications précédentes caractérisée par le fait que l'agent plastifiant est présent en une proportion comprise entre 0,1 et 80% et de préférence entre 5 et 40% en poids par rapport au poids du polymère filmogène neutralisé.

9. Composition cosmétique selon l'une quelconque des revendications précédentes caractérisée par le fait que l'agent plastifiant est choisi dans le groupe constitué par le dipropylène glycol méthyléther, le tripropylène glycol méthyléther, l'adipate de diéthyle et l'adipate de diisopropyle.

10. Composition cosmétique selon l'une quelconque des revendications précédentes, caractérisée par le fait qu'elle se présente sous forme d'une laque aérosol contenant la dispersion aqueuse telle que définie à la revendication 1, en une proportion telle que le polymère filmogène dispersé soit présent à une concentration comprise entre 2 et 30% et de préférence entre 5 et 20% en poids, en mélange avec 38 à 63% en poids d'eau et 20 à 60% en poids d'un agent propulseur par rapport au poids total de la composition.

11. Composition cosmétique selon la revendication 10, caractérisée par le fait que l'agent propulseur est l'azote, l'air comprimé, l'anhydride carbonique, le propane, le butane, l'isobutane, les dérivés fluorés, des hydrocarbures aliphatiques ou cycliques et leurs mélanges mais de préférence le diméthyléther.

12. Composition cosmétique selon l'une quelconque des revendications 1 à 9, caractérisée par le fait qu'elle se présente sous forme d'une lotion de fixation ou de mise en plis contenant la dispersion aqueuse telle que définie à la revendication 1, en une proportion telle que le polymère filmogène dispersé soit présent à une concentration comprise entre 2 et 30% et de préférence entre 5 et 20% en poids, en mélange avec 70 à 86% en poids d'eau par rapport au poids total de la composition.

13. Composition cosmétique selon l'une quelconque des revendications 1 à 9, caractérisée par le fait qu'elle se présente sous forme d'une mousse de coiffage. contenant la dispersion aqueuse telle que définie à la revendication 1, en une proportion telle que le polymère filmogène dispersé soit présent à une concentration comprise entre 2 et 15% et de préférence entre 2 et 10% en poids en mélange avec 70 à 90% en poids d'eau et 2 à 15% en poids d'un agent propulseur par rapport au poids total de la composition.

14. Composition selon l'une quelconque des revendications précédentes, caractérisée par le fait qu'elle contient en outre au moins un additif cosmétique choisi parmi : un filtre solaire, un agent anti-mousse, un agent hydratant, un humectant, un parfum, un conservateur, un colorant, un anti-oxydant, un polymère, une protéine ou une silicone.

## Claims

1. Aqueous cosmetic composition for fixing the hair, characterized in that it consists of an aqueous dispersion of:
a) particles of a film forming polymer containing carboxylic acid functions, having an average diameter of between 10 and 300 nm, the said polymer being chosen from:
(i) vinyl acetate/crotonic acid polyoxyethylenated copolymers,
(ii) vinyl acetate/crotonic acid copolymers,
(iii) vinyl acetate/crotonic acid/vinyl neodecanoate terpolymers,
(iv) N-octylacrylamide/methyl methacrylate/hydroxypropyl methacrylate/acrylic acid/tert-butylaminoethyl methacrylate copolymers,
(v) methyl vinyl ether/maleic anhydride alternating copolymers monoesterified with butanol,
(vi) acrylic acid/ethyl acrylate/N-tert-butylacrylamide terpolymers, and
(vii) polymers corresponding to the following general formula: 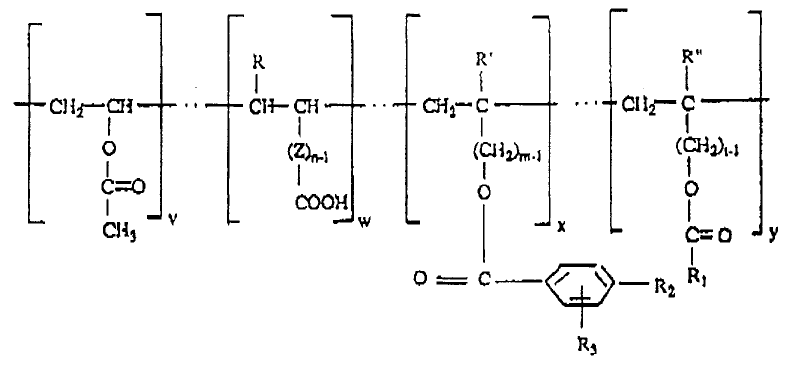 in which:
R, R' and R'', which may be identical or different, represent a hydrogen atom or a methyl radical,
m, n and t are 1 or 2,
R₁ represents a saturated or unsaturated, linear or branched alkyl radical having from 2 to 21 carbon atoms,
R₂ represents a hydrogen atom or a methyl, ethyl, tert-butyl, ethoxy, butoxy or dodecyloxy radical,
R₃ represents a hydrogen atom, an alkyl radical having 1 to 4 carbon atoms or an alkoxy radical having 1 to 4 carbon atoms,
Z represents a bivalent radical selected from the group consisting of: -CH₂-, -CH₂-O-CH₂- and -CH₂-O-(CH₂)₂-,
v represents from 10 to 91 % and preferably from 36 to 84 % by weight,
w represents from 3 to 20 % and preferably from 6 to 12 % by weight,
x represents from 4 to 60 % and preferably from 6 to 40 % by weight, and
y represents from 0 to 40 % and preferably from 4 to 30 % by weight,
v + w + x + y being equal to 100 %,
the carboxylic acid functions of the said polymer being neutralized to a degree of neutralization of between 10 and 80 % using a non-volatile monobasic agent, and
b) at least one plasticizing agent, the said agent being distributed according to its partition coefficient between the said particles and the aqueous phase.

2. Cosmetic composition according to Claim 1, characterized in that the said aqueous dispersion leads after drying to the formation of a film whose surface energy is between 30 and 55 mJ/m² and whose polarity is between 0 and 0.34 and preferably less than 0.25.

3. Cosmetic composition according to Claim 1 or 2, characterized in that the film-forming polymer containing carboxylic acid functions has an average molecular weight of between 5,000 and 700,000.

4. Cosmetic composition according to any one of the preceding claims, characterized in that the film-forming polymer containing carboxylic acid functions is neutralized using a non-volatile monobasic agent chosen from sodium hydroxide, potassium hydroxide, 2-amino-2-methylpropanol, triethanolamine, triisopropanolamine, monoethanolamine, diethanolamine, tris(2-hydroxy-1-propyl)amine, 2-amino-2-methyl-1,3-propanediol and 2-amino-2-hydroxymethyl-1,3-propanediol.

5. Cosmetic composition according to any one of the preceding claims, characterized in that, if the carboxylic acid functions of the film-forming polymer have less than 2 meq/g of carboxylic acid functions, the degree of neutralization is between 30 and 80 % and preferably between 40 and 70 %.

6. Cosmetic composition according to any one of Claims 1 to 4, characterized in that, if the carboxylic acid functions of the film-forming polymer have more than 2 meq/g of carboxylic acid functions, the degree of neutralization is between 10 and 50 % and preferably between 10 and 40 %.

7. Cosmetic composition according to any one of the preceding claims, characterized in that it contains from 1 to 40 % by weight of particles of the film-forming polymer relative to the total weight of the composition.

8. Cosmetic composition according to any one of the preceding claims, characterized in that the plasticizing agent is present in a proportion of between 0.1 and 80 % and preferably between 5 and 40 % by weight relative to the weight of the neutralized film-forming polymer.

9. Cosmetic composition according to any one of the preceding claims, characterized in that the plasticizing agent is chosen from the group consisting of dipropylene glycol methyl ether, tripropylene glycol methyl ether, diethyl adipate and diisopropyl adipate.

10. Cosmetic composition according to any one of the preceding claims, characterized in that it takes the form of an aerosol lacquer containing the aqueous dispersion as defined in Claim 1, in a proportion such that the dispersed film-forming polymer is present at a concentration of between 2 and 30 % and preferably between 5 and 20 % by weight, mixed with 38 to 63 % by weight of water and 20 to 60 % by weight of a propellent agent relative to the total weight of the composition.

11. Cosmetic composition according to Claim 10, characterized in that the propellent agent is nitrogen, compressed air, carbon dioxide, propane, butane, isobutane, fluorinated derivatives, aliphatic or cyclic hydrocarbons and mixtures thereof, but preferably dimethyl ether.

12. Cosmetic composition according to any one of Claims 1 to 9, characterized in that it takes the form of a fixing or setting lotion containing the aqueous dispersion as defined in Claim 1, in a proportion such that the dispersed film-forming polymer is present at a concentration of between 2 and 30 % and preferably between 5 and 20 % by weight, mixed with 70 to 86 % by weight of water, relative to the total weight of the composition.

13. Cosmetic composition according to any one of Claims 1 to 9, characterized in that it takes the form of a styling mousse containing the aqueous dispersion as defined in Claim 1, in a proportion such that the dispersed film-forming polymer is present at a concentration of between 2 and 15 % and preferably between 2 and 10 % by weight, mixed with 70 to 90 % by weight of water and 2 to 15 % by weight of a propellent agent, relative to the total weight of the composition.

14. Composition according to any one of the preceding claims, characterized in that it contains, in addition, at least one cosmetic additive chosen from a sunscreen agent, an antifoaming agent, a hydrating agent, a humectant, a perfume, a preservative, a colorant, an antioxidant, a polymer, a protein or a silicone.

## Patentansprüche

1. Wäßrige kosmetische Zubereitung zum Haarfestigen, dadurch gekennzeichnet, daß sie aus einer wäßrigen Dispersion (mit den folgenden Bestandteilen) besteht:
(a) aus Teilchen eines filmbildenden Polymeren mit Carbonsäurefunktionen, wobei die Teilchen einen mittleren Durchmesser aufweisen, der zwischen 10 und 300 nm beträgt, wobei das Polymer unter den folgenden ausgewählt ist:
(i) aus polyoxyethylierten Vinylacetat/Krotonsäure-Copolymeren,
(ii) Vinylacetat/Crotonsäure-Copolymeren,
(iii) Vinylacetat/Crotonsäure/Vinylneodecanoat-Terpolymeren,
(iv) N-Octylacrylamid/Methylmethacrylat/Hydroxypropylmethacrylat/Acrylsäure/tert-Butylaminoethylmethaclylat-Copolymeren,
(v) mittels Butanol monoveresterte alternierende Methylvinylether/Maleinsäureanhydrid-Copolymeren,
(vi) Acrylsäure/Ethylacrylat/N-tert-Butylacrylamid-Terpolymeren, sowie
(vii) Polymeren, die der folgenden allgemeinen Formel entsprechen: 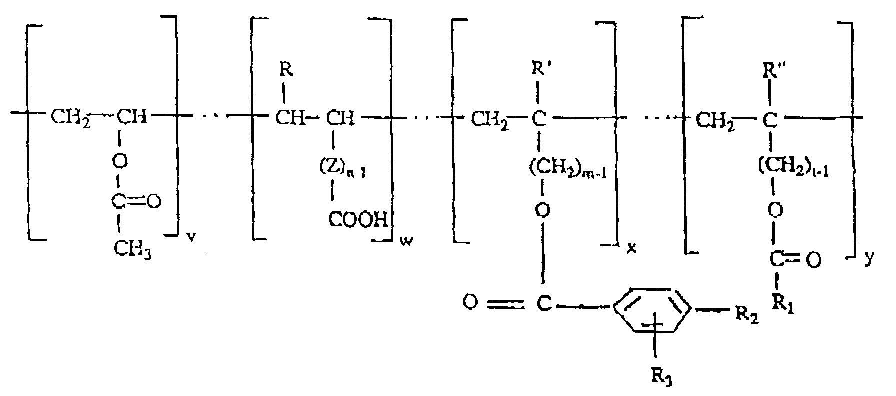 worin:
R, R' und R'', die gleich oder verschieden sind, ein Wasserstoffatom oder eine Methylgruppe bedeuten,
m, n und t 1 oder 2 betragen,
R₁ einen gerad- oder verzweigtkettigen gesättigten oder ungesättigten Alkylrest mit 2 bis 21 Kohlenstoffatomen darstellt,
R₂ ein Wasserstoffatom, eine Methyl, Ethyl-, tert-Butyl-, Ethoxy-, Butoxy- oder Dodecyloxygruppe bedeutet,
R₃ ein Wasserstoffatom, einen Alkylrest mit 1 bis 4 Kohlenstoffatomen oder einen Alkoxyrest mit 1 bis 4 Kohlenstoffatomen darstellt,
Z einen zweiwertigen Rest darstellt, der aus der aus -CH₂-, -CH₂-O-CH₂- und -CH₂-O-(CH₂)₂-bestehenden Gruppe ausgewählt ist,
v 10 bis 91 Gew.-%, vorzugsweise 36 bis 84 Gew.-% bedeutet,
w 3 bis 20 Gew.-%, vorzugsweise 6 bis 12 Gew.-% bedeutet,
x 4 bis 60 Ge.w-%, vorzugsweise 6 bis 40 Gew.-% bedeutet, und
y 0 bis 40 Gew.-%, vorzugsweise 4 bis 30 Gew.-% bedeutet,
wobei v + w + x + y zusammen 100 % ausmachen, und
die Carbonsäurefunktionen des Polymeren mittels eines monobasischen, nichtflüchtigen Mittels bis zu einem Neutralisationsgrad neutralisiert sind, der zwischen 10 und 80 % betragt, sowie
(b) aus noch mindestens einem Weichmacher besteht, wobei dieser je nach dem Aufteilungskoeffizienten zwischen diesen Teilchen und der wäßrigen Phase verteilt ist.

2. Kosmetische Zubereitung nach Anspruch 1, dadurch gekennzeichnet, daß die wäßrige Dispersion nach dem Trocknen zur Bildung eines Films führt, dessen Oberflächenenergie zwischen 30 und 55 mJ/m² und dessen Polarität zwischen 0 und 0,34, vorzugsweise weniger als 0,25 betragen.

3. Kosmetische Zubereitung nach Anspruch 1 oder 2, dadurch gekennzeichnet, daß das filmbildende Polymer mit Carbonsäurefunktionen ein durchschnittliches Molekulargewicht aufweist, das zwischen 5 000 und 700 000 beträgt.

4. Kosmetische Zubereitung nach einem der vorstehenden Ansprüche, dadurch gekennzeichnet, daß das filmbildende Polymer mit Carbonsäurefunktionen mittels eines monobasischen, nichtflüchtigen Mittels neutralisiert ist, das unter den folgenden ausgewählt ist: Natriumcarbonat, Kaliumcarbonat, 2-Amino-2-methylpropanol, Triethanolamin, Triisopropanolamin, Monoethanolamin, Diethanolamin, Tri[(2-hydroxy)-1-propyl]-amin, 2-Amino-2-methyl-1,3-propan-diol und 2-Amino-2-hydroxymethyl-1,3-propandiol.

5. Kosmetische Zubereitung nach einem der vorstehenden Ansprüche, dadurch gekennzeichnet, daß für den Fall, daß die Carbonsäurefunktionen des filmbildenden Polymers weniger als 2 mÄq/g an Carbonsäurefunktionen ausmachen, der Neutralisationsgrad zwischen 30 und 80 %, vorzugsweise zwischen 40 und 70 % beträgt.

6. Kosmetische Zubereitung nach einem der Ansprüche 1 bis 4, dadurch gekennzeichnet, daß für den Fall, daß die Carbonsäurefunktionen des filmbildenden Polymers mehr als 2 mÄq/g an Carbonsäurefunktionen ausmachen, der Neutralisationsgrad zwischen 10 und 50 %, vorzugsweise zwischen 10 und 40 % beträgt.

7. Kosmetische Zubereitung nach einem der vorstehenden Ansprüche, dadurch gekennzeichnet, daß sie 1 bis 40 Gew.-% Teilchen des filmbildenden Polymeren in bezug auf das Gesamtgewicht der Zusammensetzung enthält.

8. Kosmetische Zubereitung nach einem der vorstehenden Ansprüche, dadurch gekennzeichnet, daß der Weichmacher in einem Mengenverhältnis vorhanden ist, das zwischen 0,1 und 80 Gew.-%, vorzugsweise zwischen 5 und 40 Gew.-% in bezug auf das Gesamtgewicht des neutralisierten filmbildenden Polymers beträgt.

9. Kosmetische Zubereitung nach einem der vorstehenden Ansprüche, dadurch gekennzeichnet, daß der Weichmacher aus der aus Dipropylenglykolmethylether, Tripropylenglykolmethylether, Diethyladipat und Diisopropyladipat bestehenden Gruppe ausgewählt ist.

10. Kosmetische Zubereitung nach einem der vorstehenden Ansprüche, dadurch gekennzeichnet, daß sie in Form eines Lackaerosols vorliegt, der eine wäßrige Dispersion gemäß Definition nach Anspruch 1 in einem solchen Mengenverhältnis enthält, daß das dispergierte filmbildende Polymer in einer Konzentration vorhanden ist, die zwischen 2 und 30 Gew.-%, vorzugsweise zwischen 5 und 20 Gew.-% im Gemisch mit 38 bis 63 Gew.-% Wasser sowie 20 bis 60 Gew.-% eines Treibmittels in bezug auf das Gesamtgewicht der Zusammensetzung vorhanden ist.

11. Kosmetische Zubereitung nach Anspruch 10, dadurch gekennzeichnet, daß das Treibmittel Stickstoff, Druckluft, Kohlensäureanhydrid, Propan, Butan, Isobutan, fluorierte aliphatische oder cyclische Kohlenwasserstoffderivate sowie deren Gemische, jedoch vorzugsweise Dimethylether, ist.

12. Kosmetische Zubereitung nach einem der Ansprüche 1 bis 9, dadurch gekennzeichnet, daß sie in Form einer Festigerlotio oder Lotio zum wellenlegen vorliegt, welche die wäßrige Dispersion gemäß Definition nach Anspruch 1 in einem solchen Mengenverhältnis enthält, daß das filmbildende dispergierte Polymer in einer Konzentration vorhanden ist, die zwischen 2 und 30 Gew.-%, vorzugsweise zwischen 5 und 20 Gew.-% zusammen im Gemisch mit 70 bis 86 Gew.-% Wasser in bezug auf das Gesamtgewicht der Zusammensetzung vorhanden ist.

13. Kosmetische Zubereitung nach einem der vorstehenden Ansprüche 1 bis 9, dadurch gekennzeichnet, daß sie in Form eines Frisierschaums mit einem Gehalt an einer wäßrigen Dispersion gemäß Definition nach Anspruch 1 in einem Mengenverhältnis enthält, daß das dispergierte filmbildende Polymer in einer Konzentration vorhanden ist, die zwischen 2 und 15 Gew.-%, vorzugsweise zwischen 2 und 10 Gew.-% zusammen im Gemisch mit 70 bis 90 Gew.-% Wasser und 2 bis 15 Gew.-% Treibmittel in bezug auf das Gesamtgewicht der Zusammensetzung beträgt.

14. Zubereitung nach einem der vorstehenden Ansprüche, dadurch gekennzeichnet, daß sie zusätzlich noch mindestens einen kosmetischen Zusatzstoff enthält, der unter den folgenden ausgewählt ist: einer Sonnenfiltersubstanz, einem Antischaummittel, einem Hydratisierungsmittel, einem Feuchthaltemittel, einem Duftstoff, einem Konservierungsmittel, einem Farbstoff, einem Antioxidans, einem Polymeren, einem Protein oder einem Silikon.
